Europäisches Patentamt

(19) European Patent Office

Office européen des brevets

(11) Publication number: **0 003 015**
**B1**

(12) **EUROPEAN PATENT SPECIFICATION**

(45) Date of publication of patent specification: **29.09.82**

(21) Application number: **79300008.4**

(22) Date of filing: **03.01.79**

(51) Int. Cl.³: **G 01 N 33/48,**
**G 01 N 21/64, A 61 B 5/14**

(54) Apparatus for non-invasive detection of zinc protoporphyrin in erythrocytes.

(30) Priority: **03.01.78 US 866788**

(43) Date of publication of application:
**11.07.79 Bulletin 79/14**

(45) Publication of the grant of the patent:
**29.09.82 Bulletin 82/39**

(84) Designated Contracting States:
**DE FR GB IT**

(56) References cited:
**DE - A - 2 630 606**
**US - A - 3 536 898**
**US - A - 3 811 777**
**US - A - 3 999 062**

**Bell Laboratories Record, vol. 53 no. 6, June
1975 Murray Hill USA "Design Details at New
Lead-Poison Tester offered to Manufacturers"
Page 285.**

(73) Proprietor: **Shapiro, Howard Maurice**
**283 Highland Avenue**
**West Newton Massachusetts 02165 (US)**

(72) Inventor: **Shapiro, Howard Maurice**
**283 Highland Avenue**
**West Newton Massachusetts 02165 (US)**

(74) Representative: **Johnson, Terence Leslie et al,**
**Edward Evans & Co. Chancery House 53-64**
**Chancery Lane**
**London, WC2A 1SD (GB)**

Courier Press, Leamington Spa, England.

Apparatus for non-invasive detection of zinc protoporphyrin in erythrocytes

Technical field of the invention

The invention relates to an apparatus for the non-invasive detection of zinc protoporphyrin in erythrocytes.

Background art

It has long been recognised that the ingestion of lead bearing compounds by humans may lead to lead poisoning. If such poisoning is not detected and treated, irreversible neurological damage may result. Previously, the measurement of lead in the blood has been the primary approach for detecting lead poisoning. However, this approach is difficult and relatively expensive to implement for large scale population screening. Recently, it has been recognised that the compound zinc protoporphyrin (ZPP) accumulates in erythrocytes (red blood cells) in humans exposed to low toxic levels of lead. It has further been observed that ZPP is characterised by a red fluorescence emission spectrum (having a strong peak at 590 nm and a weak peak at 640 nm) in response to excitation with blue light (at about 420 nm). In view of these observations, protoporphyrin fluorescence detection procedures have become accepted methods of screening for lead exposure. Furthermore, such procedures have also been determined to be effective in screening for iron deficiency anaemia (such as may be due to bleeding, inadequate diet, heredity diseases) and for the detection of exposure to certain drugs, for example, the experimental anti-cancer agent cisdiamminedichloroplatinum.

Spectrophotometric apparatus can be used to study living tissue. Such apparatus is known from US—A—3 811 777 in accordance with which there is disclosed the measurement of the fluorescence from a fixed amount of living tissue, where the flow of blood through that tissue produces measurement noise. To compensate for this noise, during time periods between fluorescence measurements, a reflectance signal is generated to provide measure of the noise due to the blood flow. This has the disadvantage that different red blood cell populations are measured because of the described intermittant measurement of fluorescence. Therefore it is not possible to provide a measurement from a single blood cell population, and thus leads to inaccuracies in the results obtained.

ZPP fluorescence detection has previously been accomplished only using invasive techniques, that is, where a sample of blood is first obtained, and then analysed using the ZPP fluorescence detection technique. Such a technique is known from Bell Laboratories Record, Volume 53, Number 6, June 1975, Page 285. This type of invasive procedure requires considerable effort in terms of obtaining samples, maintaining these samples, and performing and recording the test results. Accordingly, screening of medium to large size populations is quite difficult using the prior art approaches.

Disclosure of the invention

The invention as claimed is intended to provide a remedy. It solves the problem of how to provide an apparatus for the non-invasive detection of zinc protoporphyrin (ZPP) in erythrocytes (red blood cells) in a region underlying a sample surface of the patient. By way of example, the sample surface may be skin, conjunctiva, or mucous membranes.

The advantages of the invention are that a light source may be directed to the skin of the patient. The spectrum of the source is at least partially within the fluorescence excitation spectrum of ZPP and outside the characteristic fluorescence emission spectrum of ZPP. The incident light passes through the skin and excites ZPP fluorescence in any red blood cells passing through blood vessels within the region underlying the sample surface which does not have to be p ctured as in previous invasion technique A portion of the red fluorescence emit by ZPP passes from the region, through he skin and to a detector sensitive to light in the fluorescence emission spectrum of ZPP. The detector provides a signal related to the number of red blood cells passing through the region, as well as the level of ZPP in those cells. A second detector, which is sensitive to incident light within the spectrum of the source, is also coupled to the sample surface in order to measure light reflected (or scattered) from the red blood cells in the region. The second detector provides a signal related to the number of red blood cells passing through the region. The signals from the two detectors are combined to provide a signal representative of the concentration of ZPP in the erythrocytes.

Brief description of the drawings

Two ways of carrying out the invention are described in detail below with reference to drawings which illustrate two specific embodiments, in which:—

Figure 1 is a block diagram of an exemplary apparatus according to the invention, and

Figure 2 is a block diagram of an alternative embodiment of the invention.

Best modes for carrying out the invention

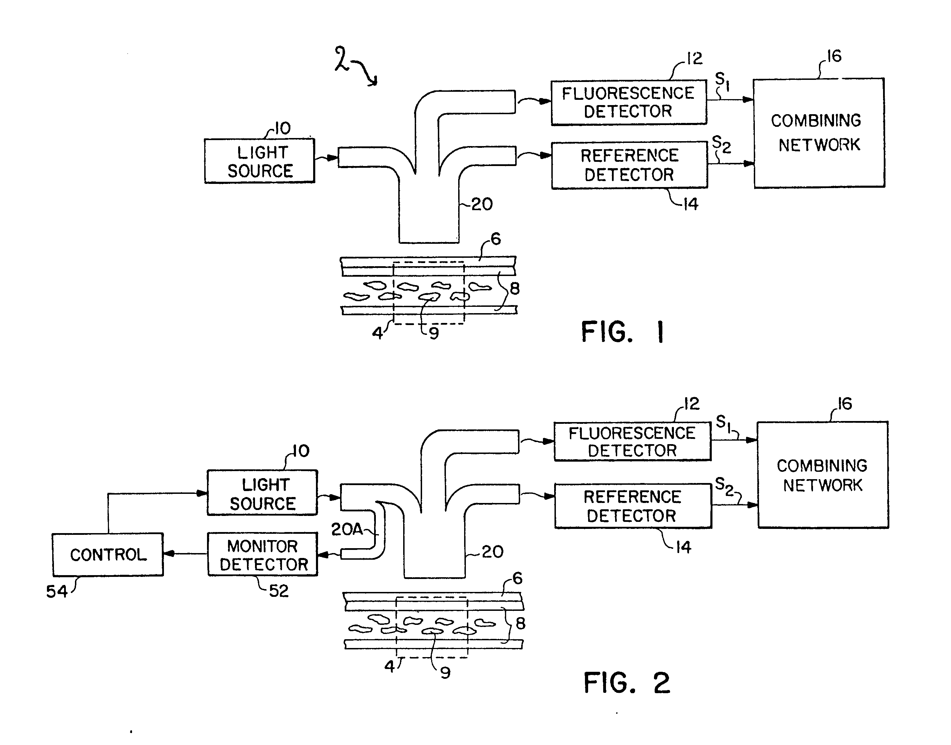

Figure 1 shows an exemplary embodiment of an apparatus 2 for the non-invasive detection of ZPP in erythrocytes in a region 4 underlying a sample surface 6 of a patient, in accordance with the invention. The illustrated region 4 which underlies the sample surface 6 includes connective tissue and a blood vessel defined by walls 8. Red blood cells (represented by reference numeral 9) are transported by the plasma flowing within the blood vessel walls 8.

The apparatus 2 includes a light source 10, including associated filters, for generating excitation light in the characteristic fluorescence excitation spectrum of ZPP. By way of example, a tungsten-halogen lamp emits sufficient light in the range 420—430 nm to serve as an adequate source for the fluorescence excitation. Alternatively, xenon or mercury or other arc lamps or blue light-emitting-diodes, or lasers operating in this spectral portion could be used in association with suitable filters. In response to light incident in its fluorescence excitation spectrum ZPP emits light with a characteristic emission spectrum exhibiting a strong peak at 590 nm and a weak peak at 640 nm.

The apparatus 2 further includes a fluorescence detector 12 for generating a signal $S_1$ representative of the intensity of light incident on that detector and in the characteristic fluorescence emission spectrum of ZPP. By way of example, the detector may take the form of a photomultiplier tube (having an S-1 or S-20 photo-cathode), or a solid state device, e.g. a phototransistor or photodiode operational amplifier combination. In conjunction with the detector, a broad-band colour or interference filter may be used to limit the detector response to wavelengths in the range 590—680 nm. In alternative embodiments, the detector 12 may be adapted to respond to incident light within relatively narrow ranges including 590 nm or 640 nm, or both.

In addition, the apparatus 2 includes a reference detector 14 for generating a signal $S_2$ representative of the intensity of light incident on that detector and the characteristic fluorescence excitation spectrum of ZPP.

In alternative embodiments, the source 10 may also generate light in some other band outside the ZPP excitation spectrum, as well as within that spectrum, and the detector 14 may generate the signal $S_2$ representative of light incident thereon in that other band.

Thus the source 10 may include a tungsten-halogen lamp and associated filter assembly adapted to emit light in the range 420—430 nm, and in addition include an infra-red LED and associated filter assembly adapted to emit light at 900 nm, i.e. at a wavelength where the level which is back scattered from a skin sample, is relatively sensitive to changes in blood flow rate but insensitive to haemoglobin oxygenation. In this example, the fluorescence detector 12 generates signal $S_1$ in the same manner as described above, while the reference detector 14 is adapted to generate signal $S_2$ representative of 900 nm light incident thereon.

In the above embodiments, the signals $S_1$ and $S_2$ from detectors 12 and 14, respectively, are applied to a combining network 16 which generates a signal representative of a concentration of ZPP in the erythrocytes within the region 4. In the present embodiment, network 16 generates a signal proportional to the ratio $S_1/S_2$.

A light guide 20 is adapted to direct the excitation light from the source 10 to the sample skin surface 6 of a patient. Guide 20 is also adapted to transfer light emitted from the sample surface to be incident on the sensors of the fluorescence detector 12 and the reference detector 14. The light guide 20 may be conventionally constructed of a fiber-optics device in combination with suitable lenses and mirrors in order to minimise errors which might arise to variations in source-to-skin distance due to motion of the patient. However, in alternative embodiments, lens configurations alone may be suitable to guide the light between the source, detectors and sample skin surface.

In operation, the excitation light from source 10 is directed to pass through surface 6 into region 4. This light is attenuated, due to scattering and absorption, as it passes through the skin and tissue, by a factor which is substantially constant for relatively short observation periods. The excitation light is also scattered or reflected and to some extent absorbed by the red blood cells passing through that region. A portion of the scattered or reflected excitation light passes to the surface 6 where it is directed to the reference detector 14. The intensity of this light incident on the detector 14 varies with the number of red blood cells within region 4. As a result of these effects, the d.c. component of reference signal $S_2$ from detector 14 varies inversely with the amount of attenuation due to the tissue, while the a.c. component of that signal is proportional to the blood flow rate. Furthermore, the portion of the excitation light which is incident upon the red blood cells in region 4 excites the ZPP in those cells. A portion of the resultant fluorescence passes to the surface 6 where it is directed to fluorescence detector 12.

The fluorescence signal $S_1$ from detector 12 is proportional to the intensity of the fluorescence from the red blood cells passing through the region 4 during the observation interval. Since the transport of fluorescent substances into and out of region 4 is primarily due to the flow of red blood cells, the a.c. component of signal $S_1$ is proportional to the ZPP in the red blood cells passing through region 4. Since the background fluorescence and attenuation factors are relatively constant, the ratio signal provided by network 16 provides a measure of the ZPP concentration in the red blood cells passing through the region 4.

In an alternative form of the invention, the combining network 16 rectifies the signal $S_1$, eliminating the d.c. component due to interfering fluorescence, and scales that rectified signal by a factor inversely proportional to the amplitude of the d.c. component of $S_2$, thereby compensating for tissue attenuation.

In practice, the intensity of illumination from source 10 may fluctuate with time. This effect may be minimised with use of a well-regulated power supply. Alternatively, the system configuration shown in Figure 2 may be utilised. This configuration is substantially the same as that shown in Figure 1, but includes a control branch for the source 10. There is a branch 20A, comprising part of the light transfer device 20, for applying a portion of the light generated by source 10 to a monitor detector 52. Detector 52 produces an output signal proportional to the intensity of the light received by way of branch 20A. A control network 54 is responsive to the signal from the detector 52 to apply a control signal to source 10 to modify the output of that light source in a manner minimising intensity fluctuations.

In these or other embodiments, compensation for blood flow fluctuations may be achieved by use of additional sources and scatter, reflectance or absorbance detectors operating at wavelength bands outside those of interest. Noise reduction may be achieved through the use of chopping or other source modulating techniques in conjunction with synchronous detection techniques.

**Claims**

1. Nor. .sive detection apparatus (2) for the detec.... of zinc protoporphyrin (ZPP) in erythrocytes (9) in a region (4) underlying a sample surface (6) of a patient, characterised by source means (10) for generating excitation light in a predetermined spectrum, said predetermined spectrum being outside the characteristic fluorescence emission spectrum of ZPP and at least a portion of said predetermined spectrum being in the characteristic fluorescence excitation spectrum of ZPP, by first detection means (12) for generating a first signal (51) representative of the intensity of light incident thereon and in said characteristic fluorescence emission spectrum of ZPP, by second detection means (14) for generating a second signal (52) representative of the intensity of reflected excitation light incident thereon within said predetermined spectrum, by transfer means (20) both for directing said excitation light to be incident on said sample surface and for directing light emitted from said sample surface to be incident simultaneously on said first and second detection means, and by circuit means (16) responsive to said first and second signals to generate a signal representative of the concentration of ZPP in said erythrocytes.

2. Apparatus according to Claim 1, characterised by third detection means (52) for generating an excitation level signal representative of the intensity of light incident thereon and in said fluorescence excitation spectrum, and by monitor transfer means (20A) for directing a predetermined portion of said excitation light to be incident on said third detection means, whereby said excitation level signal is representative of the intensity of said excitation light.

3. Apparatus according to Claim 2, characterised by source control means (54) responsive to said excitation level signal to adaptively control said source means (10) whereby the intensity of said light incident in said third detection means (52) is substantially constant.

4. Apparatus according to any preceding claim, characterised in that the circuit means (16) comprises means responsive to the first signal to generate a third signal representative of the amplitude of the a.c. component of the first signal, means responsive to the second signal to generate a fourth signal representative of the amplitude of the a.c. component of the second signal, and means responsive to the third and fourth signals to generate a fifth signal representative of the ratio of the third and fourth signals, the fifth signal representative of the concentration of ZPP in the erythrocytes.

5. Apparatus according to Claim 1, characterised in that the circuit means (16) comprises means responsive to said first and second signals to generate a ZPP signal representative of the ratio of said first and second signals, said ZPP signal being representative of the concentration of ZPP in said erythrocytes.

6. Apparatus according to any of Claims 1 to 5, characterised in that the predetermined spectrum includes at least a portion outside said characteristic excitation spectrum and in that said second detection means (14) is adapted whereby said second signal is representative of the intensity of light incident thereon and in said portion outside said characteristic excitation spectrum.

**Patentansprüche**

1. Nichtinvasives Nachweisgerät für den Nachweis von Zinkprotoporphyrin (ZPP) in Erythrozyten in einem unterhalb einer Probefläche eines Patienten befindlichen Bereich, gekennzeichnet durch ein Quellenmittel (10) zur Erzeugung von Erregungslicht in einem vorbestimmten Spektrum, wobei sich das besagte vorbestimmte Spektrum außerhalb des charakteristischen Fluoreszenzemissionsspektrums des ZPP und mindestens ein Teil des besagten vorbestimmten Spektrums innerhalb des charakteristischen Fluoreszenzerregungsspektrums des ZPP befindet, durch ein erstes Nachweismittel (12) zum Erzeugen eines ersten Signals, das für die Intensität des darauf fallenden Lichtes repräsentativ und innerhalb des besagten charakteristischen Fluoreszenzemissionsspektrums des ZPP ist, durch ein zweites Nachweismittel (14) zur Erzeugung eines zweiten Signals, das für die Intensität des darauf fallenden reflektierten Erregungslichtes innerhalb des besagten vorbestimmten Spektrums repräsentativ ist, durch ein Übertragungsmittel (20), des einerseits das besagte Erregungslicht so leitet, daß es auf die besagte Probefläche fällt und andererseits das von der besagten Probefläche emittierte Licht so steuert, daß es gleichzeitig auf das besagte erste und das besagte zweite Nachweismittel fällt, und durch ein Schaltungsmittel (16), das auf die besagten ersten und zweiten Signale anspricht, so daß ein für die Konzentration des ZPP in den besagten Erythrozyten repräsentatives Signal erzeugt wird.

2. Gerät nach Anspruch 1, gekennzeichnet durch eine dritte Nachweisvorrichtung (52) zur Erzeugung eines Anregungspegelsignals, das für die Intensität des darauf fallenden Lichtes repräsentativ ist und sich in dem besagten Fluoreszenzerregungsspektrum befindet, sowie durch ein Kontroll-Übertragungsmittel (20A), das einen vorbestimmten Teil des besagten Erregungslichtes so leitet, daß es auf das besagte dritte Nachweismittel fällt, wobei das besagte Erregungspegelsignal für die Intensität des besagten Erregungslichtes repräsentativ ist.

3. Gerät nach Anspruch 2, gekennzeichnet durch ein Quellensteuermittel (54), das auf das besagte Erregungspegelsignal anspricht, um das besagte Quellenmittel (10) adaptiv zu steuern, so daß die Intensität des besagten Lichtes, das auf das dritte Nachweismittel (52) fällt, im wesentlichen konstant ist.

4. Gerät nach einem oder mehreren der vorherstehenden Ansprüche, dadurch gekennzeichnet, daß das Schaltungsmittel (16) auf das erste Signal ansprechende Mittel zur Erzeugung eines dritten für die Amplitude der Wechselstromkomponente des ersten Signals repräsentativen Signals, auf das zweite Signal ansprechende Mittel zur Erzeugung eines vierten für die Amplitude der Wechselstromkomponente des zweiten Signals repräsentativen Signals und auf das dritte und das vierte Signal ansprechende Mittel zur Erzeugung eines fünften für das Verhältnis zwischen dem dritten und dem vierten Signal repräsentativen Signals umfaßt, wobei das fünfte Signal für die Konzentration des ZPP in den Erythrozyten repräsentativ ist.

5. Gerät nach Anspruch 1, dadurch gekennzeichnet, daß das Schaltungsmittel (16) auf das erste und das zweite Signal ansprechende Mittel zur Erzeugung eines für das Verhältnis zwischen dem ersten und dem zweiten Signal repräsentativen ZPP-Signals umfaßt, wobei das ZPP-Signal für die Konzentration des ZPP in den besagten Erythrozyten repräsentativ ist.

6. Gerät nach einem oder mehreren der Ansprüche 1 bis 3, dadurch gekennzeichnet, daß das vorbestimmte Spektrum mindestens einen Teil außerhalb des besagten charakteristischen Erregungsspektrums umfaßt, sowie dadurch, daß das besagte zweite Nachweismittel (14) so angepaßt wird, daß das besagte zweite Signal für die Intensität des darauf fallenden Lichtes repräsentativ ist und sich in dem besagten Teil außerhalb des besagten charakteristischen Erregungsspektrums befindet.

## Revendications

1. Un appareil pour la détection de protoporphyrine de zinc (PPZ) dans des erythrocytes dans une zone sous-jacente à un prélèvement d'un patient qui est caractérisé par une source (10) de production de lumière d'excitation dans un spectre établi préalablement, ce spectre établi préalablement se trouvant en-dehors du spectre caractéristique d'émission de fluorescence du PPZ et une partie au moins de ce spectre établi préalablement se trouvant à l'intérieur du spectre caractéristique d'émission de fluorescence du PPZ, par un premier dispositif de détection (12) pour la production d'un premier signal qui est caractéristique de l'intensité de lumière incidente sur celui-ci et à l'intérieur du spectre caractéristique d'émission de fluorescence indiqué ci-dessus, par un deuxième dispositif de détection (14) pour la production d'un deuxième signal caractéristique de l'intensité de l'excitation réfléchie de la lumière incidente sur celui-ci, à l'intérieur du spectre établi préalablement ci-dessus, d'un dispositif de transfert (20) d'une part pour diriger la lumière d'excitation ci-dessus pour qu'elle soit incidente sur le prélèvement indiqué précédemment, et d'autre part pour diriger la lumière émise par ce prélèvement afin qu'elle soit incidente simultanément sur la premier et sur le deuxième dispositif de détection indiqués ci-dessus, et par un circuit (16) sensible au premier et au deuxième signal indiqués ci-dessus pour la production d'un signal caractéristique de la concentration de PPZ dans les erythrocytes en question.

2. Un appareil conforme à la revendication 1 caractérisé par un troisième dispositif de détection (52) pour la production d'un signal de niveau d'excitation qui est caractéristique de l'intensité de la lumière incidente sur celui-ci et à l'intérieur du spectre d'excitation de fluorescence ci-dessus, et par un dispositif de transfert moniteur (20A) pour orienter une partie établie d'avance de la lumière d'excitation afin qu'elle soit incidente sur le troisième dispositif de détection, au moyen duquel le signal de niveau d'excitation est caractéristique de l'intensité de la lumière d'excitation indiquée ci-dessus.

3. Un appareil conforme à la revendication 2 caractérisé par un dispositif de contrôle de la source (54) qui réagit au signal de niveau d'excitation ci-dessus pour contrôler de façon à ce que l'intensité de la lumière incidente dans la troisième dispositif de détection (52) ci-dessus soit en grande partie constante.

4. Un appareil conforme à toute revendication précédente et qui est caractérisé par le fait que le circuit (16) contient un dispositif sensible au premier signal pour produire un troisième signal caractéristique de l'amplitude de la composante en courant alternatif du premier signal, un dispositif sensible au deuxième signal pour produire un quatrième signal qui est caractéristique de l'amplitude de la composante en courant alternatif du deuxième signal, et un dispositif sensible au troisième et au quatrième signal pour produire un cinquième signal qui est caractéristique de la vitesse du troisième et du quatrième signal, le cinquième signal étabt caractéristique de la concentration de PPZ dans les erythrocytes.

5. Un appareil conforme à la revendication 1, caractérisé par le fait que le circuit (16) comprend un dispositif sensible au premier et au deuxième signal pour produire un signal PPZ caractéristique de la vitesse du premier et du deuxième signal ci-dessus, le signal PPZ étant caractéristique de la concentration de PPZ dans les erythrocytes mentionnés ci-dessus.

6. Un appareil conforme aux revendications 1 à 3, caractérisé par le fait que le spectre établi préalablement comprend au moins un partie qui se trouve en-dehors du spectre d'excitation caractéristique ci-dessus, et que le deuxième dispositif de détection ci-dessus (14) est adapté pour que le deuxième signal soit caractéristique de l'intensité de la lumière incidente dessus ainsi que dans la partie, mentionnée ci-dessus, qui se trouve en-dehors du spectre d'excitation caractéristique.

FIG. 2

FIG. 1

1